# EUROPÄISCHE PATENTANMELDUNG

(11) **EP 3 384 897 A1**
(43) Veröffentlichungstag der Anmeldung: **10.10.2018**
(21) Anmeldenummer: 17164589.8
(22) Anmeldetag: 03.04.2017
(51) Int. Cl.: A61K 8/81, A61K 8/84, A61K 8/87, A61Q 5/06

(54) **KOSMETISCHE ZUSAMMENSETZUNGEN MIT SPEZIELLEN CARBODIIMIDEN FÜR HAARE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft eine Haarkosmetische Zusammensetzung, beinhaltend mindestens eine Verbindung enthaltend mindestens drei Carbodiimidgruppen aufgebaut entsprechend der Formel (I),

R⁴ - [R¹ - N=C=N]ₙ - R⁵ (I)

wobei
n für eine ganze Zahl von 3 bis 100 steht,
R¹, R⁴ und R⁵ jeweils unabhängig voneinander für lineare oder verzweigte, aliphatische oder cycloaliphatische, gegebenenfalls substituierte Gruppen mit 1 bis 100 C-Atomen steht. Weiterhin betrifft die Erfindung Verwendungen der Haarkosmetischen Zusammensetzung sowie Verfahren zu deren Anwendung.

## Beschreibung

Die vorliegende Erfindung betrifft Haarkosmetische Zusammensetzungen, im Folgenden auch nur kosmetische Zusammensetzungen genannt, enthaltend Carbodiimide, insbesondere Polycarbodiimide bzw. wässrige Dispersionen davon für die Gestaltung von Haaren deren Verwendungen sowie Verfahren zur Anwendung davon.

Zur Gestaltung und Stabilisierung vielseitiger Frisuren werden Produkte eingesetzt, die als Haarfestiger bekannt sind. Haarfestiger gibt es meistens in Form von Schaumfestigern oder Haarsprays, wobei sie sich in ihrer Zusammensetzung kaum unterscheiden. Schaumfestiger werden auf das feuchte Haar als Hilfsmittel zur Modellierung der Frisur aufgetragen. Im Gegensatz hierzu, werden Haarsprays auf trockene fertig gestylte Haare zur Fixierung der Frisur aufgebracht.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh-oder Schäumvorrichtungen versprühbare Präparate vor, die aus einer alkoholischen oder wäßrigalkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden.

Eine kosmetische Zusammensetzung wie die der Erfindung kann der Fixierung von Gestaltungen der Haare dienen, aber ggf. zusammen mit Zusatzstoffen auch der dekorativen, insbesondere farblichen Gestaltung der des Haars, insbesondere der Augenlider und der Augenbrauen. Der dekorative Effekt wird dabei bevorzugt durch die erfindungsgemäße Zusammensetzung länger haltbar gemacht, gegen Anschmutzung geschützt und wasserbeständiger gemacht. Die erfindungsgemäße kosmetische Zusammensetzung kann beispielsweise mindestens Bestandteil eines Haarfärbemittels, eines Haarfestigers, eines Haarshampoos, einer Wimperntusche oder anderer Haarstylingprodukte sein. Die vorstehend genannte Liste von dekorativen Produkten ist selbstverständlich nicht limitierend.

Die Verbraucher wünschen sich bei Anwendung kosmetischer Formulierungen naturgemäß einen langanhaltenden kosmetischen Effekt. Insbesondere erwarten die Verbraucher eine gute Beständigkeit gegenüber Wasser, wie während des Badens oder beim Duschen, Tränen oder Schweiß wie insbesondere während sportlicher Aktivitäten.

Zur Verbesserung der Beständigkeit von kosmetischen Produkten gegenüber Wasser, Tränen oder Schweiß (oft Wasserfestigkeit genannt) werden häufig filmbildende Polymere eingesetzt. Als filmbildende Polymere werden bevorzugt Polymere auf Basis von Acrylaten oder Vinylpyrrolidone gewählt. Die Nachteile solcher filmbildenden Polymere sind dem Fachmann bekannt. Die Acrylatpolymere bilden nämlich harte und spröde Filme. Daraus resultiert ein unangenehmes Gefühl beim Tragen des Produktes. Wegen des klebrigen Gefühls, können die Vinylpyrrolidone nur in begrenzten Konzentrationen eingesetzt werden.

Die Anwendung von Polyurethandispersionen ist in der kosmetischen bzw. dekorativen Kosmetik bekannt. Die EP 1010418 beschreibt den Einsatz einer wässrigen Polyurethandispersion in einer wachs-freien Mascara-Zusammensetzung, jedoch nicht in Kombination mit Carbodiimiden. Monomere Carbodiimide wurden bereits zur Behandlung von Haaren beschrieben. So beschreibt die DE 1 617 710 A die Verwendung von Carbodiimiden zur Verbesserung der histologischen Eigenschaften von Haaren nach dem Färben. Hierzu werden Mono- und dicarbodiimide verwendet, die wasserlöslich sind. Eine langanhaltende Wirkung von Carbodiimiden auf Haaren wird hier nicht beschrieben.

Tragekomfort, insbesondere verringerte Klebrigkeit, Beständigkeit, insbesondere Wasserfestigkeit und verbessertes Benetzungsverhalten z.B. zur Verbesserung des Anschmutzverhaltens, insbesondere in Kombination mit guter Kompatibilität mit weiteren kosmetischen Mitteln der kosmetischen Zusammensetzungen aus dem Stand der Technik geben somit noch Raum für Verbesserungen.

Die vorliegende Erfindung hat daher als eine Aufgabe, mindestens einen Nachteil des Standes der Technik zumindest zu einem Teil zu verringern.

Weiterhin ist es eine Aufgabe der Erfindung, eine kosmetische Zusammensetzung bereitzustellen, welche einen hohen Tragekomfort, insbesondere eine verringerte Klebrigkeit, eine hohe Beständigkeit, insbesondere Wasserfestigkeit und verbesserte Benetzungseigenschaften aufweist. Darüber hinaus sollten die erfindungsgemäß verwendeten wässrigen Dispersionen eine vergleichsweise geringe Viskosität aufweisen, so dass sie sich leicht in kosmetische Zusammensetzungen für diverse Zwecke, z.B. zur Erreichung von dekorativen Effekten, aber auch strukturerhaltende Zwecke, einarbeiten lassen.

Eine Aufgabe der Erfindung war es, eine kosmetische Zusammensetzung, insbesondere eine Haarkosmetische Zusammensetzung bereitzustellen, die bei guter Strukturfixierung des Haares eine angenehme Haptik auf dem Haar aufweist, insbesondere sich nicht klebrig oder klumpig anfühlt.

Es war weiterhin eine Aufgabe der Erfindung, verschiedene Verwendungen der erfindungsgemäßen Zusammensetzungen zur Strukturerhaltung von in Form gebrachtem Haar bereitzustellen.

Weiterhin war es eine Aufgabe der Erfindung, verschiedene Verwendungen der erfindungsgemäßen Zusammensetzung zur Strukturerhaltung von in Form gebrachtem Haar bereitzustellen, wobei das behandelte Haar insbesondere in Kontakt mit Wasser, Shampoo oder beidem seine Struktur möglichst lange beibehält.

Eine weitere Aufgabe bestand darin ein kosmetisches Verfahren bereitzustellen, das es ermöglicht einen strukturerhaltenden Effekt auf in Form gebrachtes Haar zu erzielen, wobei das behandelte Haar insbesondere in Kontakt mit Wasser, Shampoo oder beidem seine Struktur möglichst lange beibehält.

Mindestens eine der Aufgaben wird überraschend gelöst durch eine Haarkosmetische Zusammensetzung, beinhaltend mindestens eine Verbindung enthaltend mindestens drei Carbodiimidgruppen aufgebaut entsprechend der Formel (I),

R⁴-[R¹-N=C=N]ₙ-R⁵ (I)

wobei
n für eine ganze Zahl von 3 bis 100, bevorzugt von 3 bis 50, oder bevorzugt von 3 bis 20, oder bevorzugt von 3 bis 10 steht,
R¹ und R⁵ jeweils unabhängig voneinander für lineare oder verzweigte, aliphatische oder cyclo-aliphatische, gegebenenfalls substituierte Gruppen mit 4 bis 100, oder bevorzugt von 4 bis 50, oder bevorzugt von 4 bis 20 C-Atomen steht.
R⁴ für lineare oder verzweigte, aliphatische oder cyclo-aliphatische, gegebenenfalls substituierte Gruppen mit 1 bis 100, oder bevorzugt von 1 bis 50, oder bevorzugt von 1 bis 20 C-Atomen steht.

Als Substituenten der Reste R⁴ und/oder R⁵ kommen im Rahmen der Erfindung zahlreiche organische Gruppen in Frage, beispielsweise Alkyl-, Cycloalkyl-, Aryl-, Alkoxy, Halogen-, Ether-, Thioether-, Thiourethan-, Disulfid-, Sulfoxid-, Sulfon-, Sulfonat-, Amino-, Aldehyd-, Keto-, Carbonsäureester-, Carbonsäure-, Carbonat-, Carboxylat-, Cyano-, Alkylsilan- und Alkoxysilangruppen, Urethangruppen, Allophanatgruppen, Biuretgruppen, Harnstoffgruppen sowie Carboxylamidgruppen. Insbesondere ist ein Teil, auch Substituent genannt, der Reste R¹, R⁴ oder R⁵, bevorzugt der Reste R⁴ und/oder R⁵ ausgewählt aus der Gruppe bestehend aus einer Polyethylenglycolgruppe, einer Polypropylengruppe, einer Methoxygruppe, einer Ethoxygruppe, einer Butoxygruppe, einer Methoxpolyethylenglycolgruppe, einer Urethangruppe, einer Alkylgruppe, einer Cycloalkylgruppe oder einer Kombination aus mindestens zwei hieraus.

Weiterhin bevorzugt weist mindestens einer der Reste R¹, R⁴ oder R⁵, bevorzugt die Reste R⁴ und/oder R⁵ sowohl eine Alkyl- als auch eine Cycloalkylgruppe auf. Darüber hinaus ist es bevorzugt, dass mindestens einer, besonders bevorzugt beide der Reste R⁴ oder R⁵, mindestens eine der Gruppen, bevorzugt mindestens zwei der Gruppen ausgewählt aus der Gruppe bestehend aus einer Urethangruppe, einer Polyethylenglycolgruppe (PEG), einer Polypropylengruppe (PPG), einer Methoxygruppe, einer Ethoxygruppe, einer Alkylgruppe und einer Cycloalkylgruppe oder einer Kombination aus mindestens zwei hiervon aufweist. Insbesondere ist es bevorzugt, dass die Reste R⁴ und/oder R⁵ eine Alkylgruppe, eine Cycloalkylgruppe, eine Urethangruppe, eine PEG-Gruppe und eine Methoxygruppe, bevorzugt genau in dieser Reihenfolge aufweisen. Bevorzugt enthalten beide Reste R⁴ und/oder R⁵ zumindest eine Urethangruppe sowie zumindest eine Gruppe, die für einen von einem C₁ bis C₃₀ Alkohol oder einem C₁ bis C₃₀ Monoalkoxyethylenglykol abgeleiteten Rest stehen.

Bei der Verbindung, enthaltend mindestens drei Carbodiimidgruppen, im Folgenden auch Polycarbodiimid genannt, handelt es sich bevorzugt um wasserlösliche oder wasserdipergierbare Verbindungen.

Das Polycarbodiimid, das in der erfindungsgemäßen Zusammensetzung enthalten ist hat bevorzugt ein Molekulargewicht M_{w} (bestimmt mittels GPC) in einem Bereich von 300 bis 500 000 g/mol, oder bevorzugt in einem Bereich von 500 bis 300 000 g/mol, oder bevorzugt in einem Bereich von 1000 bis 200 000 g/mol.

Ein bevorzugtes Verfahren zur Herstellung von wässrigen Dispersionen von Polycarbodiimiden, umfasst wenigstens einen Schritt, wobei in dem wenigstens einen Schritt wenigstens ein aliphatisches oder cycloaliphatisches Polyisocyanat bei einer Temperatur im Bereich von 160 bis 230 °C in Gegenwart von Carbodiimidisierungskatalysator zu einem Polycarbodiimid mit einer mittleren Funktionalität von größer 3, bevorzugt von 3 bis 100, oder bevorzugt von 3 bis 50, oder bevorzugt von 3 bis 20, oder bevorzugt von 3 bis 10 Carbodiimideinheiten umgesetzt wird. Bevorzugt werden die Reaktionsgase zeitweise oder permanent abgeführt. Bevorzugt beträgt die Menge an Carbodiimidisierungskatalysator 50 bis 3000 ppm, bezogen auf die molare Menge an Polyisocyanat.

Im Sinne der vorliegenden Erfindung bedeutet die mittlere Funktionalität von Carbodiimideinheiten die durchschnittliche Anzahl der Carbodiimideinheiten. Die mittlere Funktionalität kann auch eine gebrochene Zahl sein. Die mittlere Funktionalität beträgt bevorzugt 3 bis 50, oder bevorzugt 3 bis 20, oder bevorzugt 3 bis 10. Je höher die Funktionalität ist, desto niedriger ist die Dispergierbarkeit des hydrophilierten Polycarbodiimids in Wasser.

Bevorzugt wird bei der Herstellung des Polycarbodiimids ein aliphatisches bzw. cycloaliphatisches Polyisocyanat aus der Gruppe bestehend aus Methylendiisocyanat, Dimethylendiisocyanat, Trimethylendiisocyanat, Tetramethylendiisocyanat, Pentamethylendiisocyanat, Dipropyletherdiisocyanat, 2,2-Dimethylpentandiisocyanat, 3-Methoxyhexandiisocyanat, Octamethylendiisocyanat, 2,2,4-Trimethylpentandiisocyanat, Nonamethylendiisocyanat, Decamethylen-diisocyanat, 3-Butoxyhexandiisocyanat, 1,4-Butylenglykoldipropyletherdiisocyanat, Thiodihexyldiisocyanat, Metaxylylendiisocyanat, Paraxylylendiisocyanat, Tetramethylxylylendiisocyanat, 4,4'-Dicyclohexylmethandiisocyanat (H12MDI), Isophorondiisocyanat (IPDI), Hexamethylendiisocyanat (HDI), hydriertes Xylylendiisocyanat (H6XDI), 1,12-Diisocyanatdodecan (DDI), Norbornandiisocyanat (NBDI) und 2,4-Bis(8-isocyanatoctyl)-1,3-dioctylcyclobutan (OCDI) oder einer Mischung aus mindestens zwei hieraus ausgewählt. Besonders bevorzugt sind Isophorondiisocyanat (IPDI), Hexamethylendiisocyanat (HDI), hydriertes Xylylendiisocyanat (H₆XDI), 4,4'-Dicyclohexylmethandiisocyanat (H12MDI). Ganz besonders bevorzugt ist das cycloaliphatische Polyisocyanat 4,4'-Dicyclohexylmethandiisocyanat (H12MDI).

Bevorzugt ist der Carbodiimidisierungskatalysator eine Organophosphorverbindung, besonders bevorzugt Organophosphorverbindungen ausgewählt aus der Gruppe bestehend aus Phos-phanoxid, Phospholanoxid und Phospholenoxid, sowie deren Sulfo- und Iminoanaloga. Die Phosphan-, Phospholen- und Phospholan-Oxide, -Sulfide sowie -Iminoderivate können unter anderem aus entsprechenden Vorgängern mit dreiwertigem Phosphor wie Phosphanen, Phospholanen und Phospholenen in situ generiert werden. Bevorzugt wird das Phospholenoxid aus der Gruppe bestehend aus 3-Methyl-1-phenyl-2-phospholen-1-oxid, 3-Methyl-1-ethyl-2-phospholen-1-oxid, 1,3-Dimethyl-2-phospholen-1-oxid, 1-Phenyl-2-phospholen-1-oxid, 1-Ethyl-2-phospholen-1-oxid, 1-Methyl-2-phospholen-1-oxid ausgewählt. Weitere geeignete Katalysatoren sowie bevorzugte Ausführungsformen des Verfahrens für die Herstellung des Polycarbodiimids sind in der WO 2011/120928 A2 beschrieben

Ein bevorzugtes Verfahren zur Herstellung von wässrigen Dispersionen von Polycarbodiimid umfasst die Schritte:
a) Umsetzen von wenigstens einem aliphatischem oder cycloaliphatischem Polyisocyanat bei einer Temperatur im Bereich von 160 bis 230 °C in Gegenwart von 50 bis 3000 ppm (ppm = part per million, 1 ppm = 0.0001%, molarer Anteile) Carbodiimidisierungskatalysator bezogen auf die molare Menge an Polyisocyanat zu einem Polycarbodiimid, bevorzugt mit einer mittleren Funktionalität von 3 bis 20 Carbodiimideinheiten umgesetzt wird, wobei die Reaktionsgase zeitweise oder permanent gezielt aus dem Reaktionsmedium abgeführt werden,
b) Umsetzen des in Schritt a) erhaltenen Polycarbodiimids mit wenigstens einer hydrophilen Verbindung, die mindestens gegenüber Isocyanat- und/oder Carbodiimidgruppen reaktionsfähige Gruppe trägt, zum Beispiel aber nicht darauf beschränkt, ausgewählt aus der Gruppe bestehend aus Polyethoxymonoolen, Polyethoxydiolen, Polyethoxypolypropoxy-monoolen, Polyethoxypolypropoxydiolen, Polyethoxymonoaminen, Polyethoxydiaminen, Polyethoxypolypropoxymonoaminen, Polyethoxypolypropoxydiaminen, Hydroxyalkylsulfonaten, Aminalkylsulfonaten, Polyethoxymono- und -dithiolen, Polyethoxymono- und - dicarbonsäuren, Mono- und Dihydroxycarbonsäuren bzw. deren Salzen zu einem hydrophilierten Carbodiimid,
   gegebenenfalls weiteres Umsetzen der nicht abreagierten Isocyanatgruppen mit weiteren gegenüber Isocyanatgruppen reaktionsfähigen Verbindungen, wie zum Beispiel mit Wasser, Alkoholen, Thiolen, Aminen, Mineral- und Carbonsäuren und
c) Dispergieren der in Schritt b) erhaltenen Verbindung in Wasser.

Bevorzugt erfolgt die Umsetzung des in Schritt a) erhaltenen, Isocyanat-Gruppen enthaltenden Polycarbodiimids gemäß der vorliegenden Erfindung so, dass 10 bis 70 Molprozent (mol.-%) der im Polycarbodiimid vorhandenen Isocyanat-Gruppen mit mindestens einer hydrophilen Verbindung als Teilschritt b1) des Schritts b) umgesetzt werden, wobei die hydrophile Verbindung ausgewählt ist aus der Gruppe bestehend aus Polyethoxymonoolen, Polyethoxydiolen, Polyethoxypolypropoxymonoolen, Polyethoxypolypropoxydiolen, Polyethoxymono-aminen, Polyethoxydiaminen, Polyethoxypolypropoxymonoaminen, Polyethoxypoly-propoxydiaminen, Hydroxyalkylsulfonaten, Aminalkylsulfonaten, Polyethoxymono- und -dithiolen, Polyethoxymono-und -dicarbonsäuren. Im Teilschritt b2) des Schritts b) werden 30 bis 90 mol.-% der verbleibenden Isocyanat-Gruppen dann mit mindestens einer gegenüber Isocyanatgruppen reaktionsfähigen Verbindung umgesetzt, zum Beispiel, Polyethoxymonoolen, Polyethoxydiolen, Polyethoxypolypropoxymonoolen, Polyethoxypolypropoxydiolen, Polyethoxymonoaminen, Polyethoxydiaminen, Polyethoxypolypropoxymonoaminen, Polyethoxypolypropoxydiaminen, Hydroxyalkylsulfonaten, Aminalkylsulfonaten, Polyethoxymono- und -dithiolen, Polyethoxy-mono-und -dicarbonsäuren, Wasser, C₁ bis C₃₀ Alkoholen, C₁ bis C₃₀ Thiolen, Aminen, Mi-neral- und Carbonsäuren.

Bevorzugt erfolgt die Umsetzung der Polycarbodiimide in Teilschritt b1) des Schritts b) mit wenigstens einer Verbindung ausgewählt aus der Gruppe der Verbindungen entsprechend der allgemeinen Formel (III):

R⁶-O-(CH₂-CH₂-O)ₘ-H (III)

mit R⁶ = C₁ bis C₃₀ Alkyl oder Acylgruppe und m = 4 bis 30
besonders bevorzugt mit wenigstens einer Verbindung ausgewählt aus der Gruppe entsprechend der Formel (III), wobei R⁶ eine Methylgruppe ist und m = 10 bis 30.

Ganz besonders bevorzugt ist Monomethoxypolyethylenglykol mit m = 15-20.

Bevorzugte C1 bis C30 Alkohole, die zu einer Weiterreaktion der im Polycarbodiimid vorhan-denen und mit den hydrophilen Verbindungen nicht vollständig umgesetzten Isocyanatgruppen in Teilschritt b2) des Schritts b) verwendet werden können, sind zum einen Wasser, niedermolekulare Monoalkohole oder auch Diole mit einem Molekulargewicht bevorzugt von 32 bis 500, besonders bevorzugt von 62 bis 300 g/mol. Ganz besonders bevorzugt werden kurzkettige Monoalkohole also verzweigte oder unverzweigte Monoalkohole mit 1 bis 30 C-Atomen wie Methanol, Ethanol, Propanol, 1-Butanol, 1-Pentanol, 1-Hexanol, Cyclohexanol, Cyclohexylmethanol, 2-Ethylhexanol, Dodecanol, Stearylalkohol oder Oleylalkohol, deren Gemische untereinander und Gemische deren Isomere sowie kurzkettige Dialkohole mit 2 bis 60 C-Atomen wie 1,4-Butandiol, 1,5-Pentandiol, 1,6-Hexandiol, Cyclohexandiol, Cyclohexandimethanol, 1,8-Octandiol, 1,9-Nonadiol, 1,10-Decandiol, 1,11-Undecandiol, 1,12-Dodecandiol, 1,13-Tridecandiol, Tricyclodecandimethanol, deren Gemisch untereinander sowie Gemische deren Isomere verwendet.

Die Reihenfolge der Teilschritte b1) und b2) des Schritts b) kann so festgelegt sein, dass Teil-schritt b1) vor Teilschritt b2), beide gleichzeitig oder in der umgekehrten Reihenfolge erfolgen kann.

Optional wird die wässrige Dispersion von hydrophiliertem Polycarbodiimid in einem Schritt d) auf einen pH-Wert im Bereich von 7 bis 12 (bei 23°C), besonders bevorzugt im Bereich von 8 bis 11, eingestellt. Dafür können Aminlösungen, Laugen und herkömmliche Pufferlösungen verwendet werden.

Die mittlere Teilchengröße d50-Wert der in Wasser dispergierten Polycarbodiimidpartikel liegt üblicherweise im Bereich von 5 - 500 nm, bevorzugt von 15 bis 200 nm, besonders bevorzugt von 25 bis 100 nm. Die mittlere Teilchengröße d50 ist der Durchmesser, oberhalb und unterhalb dessen jeweils 50 Gew.-% der Teilchen liegen. Er kann mittels Ultrazentrifugenmessung (W. Scholtan, H. Lange, Kolloid. Z. und Z. Polymere 250 (1972), 782-796) bestimmt werden. Die mittleren Teilchengrößen und Teilchengrössenverteilungen können auch über Lichtstreumethoden bestimmt werden, die allerdings weniger präzise sind, jedoch mit den Ultrazentrifugationsmessungen recht gut korrelieren können, sofern keine polymodalen oder sehr breiten Teilchengrößenverteilungen vorliegen.

In einer bevorzugten Ausführungsform der Haarkosmetischen Zusammensetzung weist die Verbindung, enthaltend mindestens drei Carbodiimidgruppen, die allgemeine Formal (II) auf: 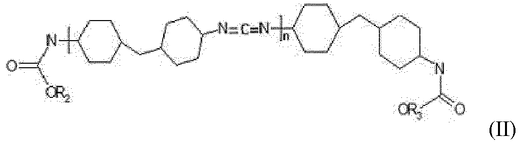 worin R₂ und R₃, unabhängig voneinander, für einen von einer Verbindung ausgewählt aus der Gruppe bestehend aus einem Monoalkoxy-poly(ethylenglykol) gemäß der allgemeinen Formel (III)

R⁶-O-(CH₂-CH₂-O)ₘ-H (III)

mit R⁶ = C₁ bis C₃₀ Alkyl oder Acylgruppe und m = 4 bis 30
und einem von C₁ bis C₃₀ Alkohol oder einem C₁ bis C₃₀ Monoalkoxyethylenglykol abgeleiteten Rest stehen.

Die wässrigen Polycarbodiimid-Dispersionen und/oder Lösungen, hergestellt nach dem oben beschriebenen Verfahren, haben üblicherweise einen Feststoffgehalt von 10 bis 80 Gew.%, bevorzugt von 20 bis 60 Gew.-% und besonders bevorzugt von 30 bis 50 Gew.%.

In einer bevorzugten Ausführungsform der Haarkosmetischen Zusammensetzung enthält die Zusammensetzung die Verbindung enthaltend das Carbodiimid, auch Polycarbodiimid genannt, der Formel (I) oder (III) in einem Bereich von 0,01 bis 50 Gew.-%, bevorzugt in einem Bereich von 0,05 bis 30 Gew-%, oder bevorzugt in einem Bereich von 0,1 bis 20 Gew.-%, oder bevorzugt in einem Bereich von 0,5 bis 10 Gew.-%, bezogen auf das Gesamtgewicht der kosmetischen Zusammensetzung. Je nach Anwendung der kosmetischen Zusammensetzung beispielsweise in Haaranwendungsprodukten, können die bevorzugten Mengen an Polycarbodiimiden stark variieren. Als Haaranwendungsprodukte werden Produkte wie Haarstyling-, Haarpflege- oder Haarfärbeprodukte verstanden. Darüber hinaus kann die kosmetische Zusammensetzung auch kolorierende Substanzen beinhalten und eine Wimperntusche, ein Mascara, ein Shampoo, ein Festiger, ein Färbemittel, wobei dies jeweils als Spray, Lotion, Creme, Schaums, Lösung, Emulsion oder Wachs vorliegen kann sein oder Bestandteil von einem solchen sein. Die kosmetische Zusammensetzung enthält das Polycarbodiimid bevorzugt in einem Bereich von 0,1 bis 10 Gew.-%, oder bevorzugt in einem Bereich von 0,2 bis 8 Gew.-%, oder bevorzugt in einem Bereich von 0,5 bis 5 Gew.-%, bezogen auf das Gesamtgewicht der kosmetischen Zusammensetzung, beispielsweise in Shampoos. Die kosmetische Zusammensetzung enthält das Polycarbodiimid bevorzugt in einem Bereich von 0,5 bis 30 Gew.-%, oder bevorzugt in einem Bereich von 1 bis 20 Gew.-%, oder bevorzugt in einem Bereich von 2 bis 15 Gew.-%, bezogen auf das Gesamtgewicht der kosmetischen Zusammensetzung, in Haarfärbeprodukten.

In einer bevorzugten Ausführungsform der Haarkosmetischen Zusammensetzung beinhaltet die Zusammensetzung weiterhin mindestens eine der folgenden Komponenten:
K1. mindestens eine kosmetisch aktive Substanzen und/oder ein oder mehrere kosmetische Hilfssubstanzen,
K2. mindestens ein Lösungsmittel oder Verdünner,
K3. mindestens einen Filmbildner.

Die erfindungsgemäße Zusammensetzung enthält bevorzugt ein oder mehrere kosmetische Hilfssubstanzen, die in der Kosmetik üblich sind, wie Antioxidantien, Lichtschutzmittel und/oder andere Hilfs- und Zusatzmittel wie beispielsweise Emulgatoren, Benetzungsmittel wie grenzflächenaktive Stoffe, Weichmacher wie Glycerin, Glykol und Phthalester- und ether, Entschäumer, Verdicker und Rheologiemodifizierer, Geliermittel Antiklebmittel, Tenside, Wirkstoffe, Feuchthaltemittel, Füllstoff, UV-Filter, Filmbildner, Lösemittel, Koaleszenzmittel, Aromastoffe, Geruchsabsorber, Riechstoffe und Parfüms, Gelbildner und/oder andere Polymerdispersionen wie beispielsweise Dispersionen auf Basis von Polyacrylaten, Pigmente, Farbstoffe, antikorrosive Mittel, Neutralisationsmittel, Verlaufsmittel und/oder Thixotropiemittel, Geschmeidigkeitsmittel, Konservierungsmittel, Proteine und Derivate davon, Aminosäuren, Vitamine, Trübungsmittel, Stabilisatoren, Sequestierungsmittel, Komplexbildner, Perlglanzmittel, ästhetische Verstärker, Fettsäuren, Fettalkohole, Triglyceride, botanische Extrakte und Klärhilfsmittel. Die Mengen der verschiedenen Zusatzstoffe sind dem Fachmann für den einzusetzenden Bereich bekannt und liegen beispielweise im Bereich von 0 bis 25 Gew.-%, bevorzugt von 0 bis 15 Gew.-%, oder bevorzugt von 0,001 bis 15 Gew.-%, oder bevorzugt für jeden einzelnen Zusatzstoff jeweils in einem Bereich von 0,001 bis 5 Gew.-%, oder jeweils bevorzugt von 0,01 bis 3 Gew.-% bezogen auf das Gesamtgewicht der Zusammensetzung.

Die erfindungsgemäßen kosmetischen Zusammensetzungen können einen oder mehrere Emulgatoren bzw. oberflächenaktive Mittel enthalten.

So enthalten erfindungsgemäße Öl-in-Wasser-Emulsionen (O/W) bevorzugt mindestens einen Emulgator mit einem HLB-Wert > 7 und gegebenenfalls einen Coemulgator.

Vorteilhaft werden die folgenden nichtionischen Emulgatoren eingesetzt:
a) Partialfettsäureester und Fettsäureester mehrwertiger Alkohole und deren ethoxylierte Derivate (z. B. Glycerylmonostearat, Sorbitanstearat, Glyceryl-Stearyl-Citrat, Sucrose-Stearat)
b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/C₁₀₋₃₀-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

Die kosmetischen Zusammensetzungen enthalten bevorzugt Verdicker, insbesondere der Wasserphase beispielsweise in einer O/W-Zusammensetzung. Vorteilhafte Verdicker sind:
- Vernetzte oder nichtvernetzte Acrylsäure- oder Methacrylsäure-Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.

- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol^{®} und Guargummi, wie die unter der Bezeichnung Jaguar^{®} HP105 von der Firma RHODIA erhältlichen Produkte.

Ganz besondere vorteilhafte Verdicker sind auch vernetzte Homopolymere von Methacrylsäure oder Acrylsäure, die von der Firma Lubrizol unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopol^{®} 2984, Carbopol^{®} 5984 und Carbopol^{®} Ultrez 10, von der Firma 3V unter den Bezeichnungen Synthalen^{®} K, Synthalen^{®} L und Synthalen^{®} MS und von der Firma PROTEX unter den Bezeichnungen Modarez^{®} V 1250 PX, Modarez^{®} V2000 PX, Viscaron^{®} A1600 PE und Viscaron^{®} A700 PE im Handel erhältlich sind.

Ganz besondere vorteilhafte Verdicker sind vernetzte Copolymer von Acrylsäure oder Methacrylsäure und einem C₁₀₋₃₀-Alkylacrylat oder C₁₀₋₃₀-Alkylmethacrylat und Copolymere von Acrylsäure oder Methacrylsäure und Vinylpyrrolidon. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma ISP unter den Bezeichnungen Ultrathix^{®} P-100 (INCI : Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCI: Ammonium Acryloyldimethyltaurate/VP Copolymer) erhältlich.

Falls die Verdicker eingesetzt werden, sind die Verdicker im Allgemeinen in einer Konzentration von etwa 0 % bis 2 Gew.-% vorzugsweise 0 % bis 1 Gew.-% oder bevorzugt in einem Bereich von 0,01 bis 1 Gew.-% bezogen auf das Gesamtgewicht der erfindungsgemäßen Zusammensetzung vorhanden.

Die bevorzugten Lösungsmittel als Komponente K2 sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren Gemische.

Der Mengenanteil des Lösungsmittels oder der Lösungsmittel in der erfindungsgemäßen Zusammensetzung kann beispielsweise im Bereich von 0 bis 25 Gew.-% und bevorzugt 0 bis 15 Gew.-%, bezogen auf das Gesamtgewicht der Zusammensetzung, liegen.

Die erfindungsgemäßen Zusammensetzungen können des weiteren ein Treibgas enthalten.

Bevorzugte Treibgase sind Kohlenwasserstoffe wie Propan, Isobutan und n-Butan sowie deren Mischungen. Druckluft, Kohlendioxid, Stickstoff, Stickstoffdioxid und Dimethylether sowie Mischungen all dieser Gase können ebenfalls verwendet werden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte. Dabei handelt es sich, insbesondere Fluorkohlenwasserstoffe und Fluorchlorkohlenwasserstoffe (FCKW) wie z.B. 1,2-difluoroethan (Treibmittel 152 A).

Bevorzugt sind in der kosmetischen Zusammensetzung des weiteren haarpflegende Wirkstoffe enthalten. Als Pflegestoffe können bevorzugt cyclische Polydimethylsiloxane (Cyclomethicone) oder Silikontenside (Polyethermodifizierte Siloxane) vom Typ Dimethicone Copolyol oder Simethicon zum Einsatz kommen. Cyclomethicone werden u. a. unter der Handelsbezeichnungen Abil^{®} K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil^{®} DM 6031 von der Firma Wacker angeboten.

### Tenside

Die erfindungsgemäßen Zusammensetzungen können auch Tenside enthalten, die aus der Gruppe von anionischen, kationischen, nichtionischen und/oder amphoteren Tensiden ausgewählt werden.

Vorteilhafte anionische Tenside im Sinne der vorliegenden Erfindung sind:
- Acylaminosäuren und deren Salze, wie Acylglutamate, insbesondere Natriumacylglutamat und Sarcosinate, beispielsweise Myristoyl Sarcosin, TEA-Lauroyl Sarcosinat, Natriumlauroylsarcosinat und Natriumcocoylsarkosinat,
- Sulfonsäuren und deren Salze, wie Acylisethionat, beispielsweise Natrium- oder Ammoniumcocoylise-thionat, Sulfosuccinate, beispielsweise Dioctylnatriumsulfosuccinat, Dinatriumlaurethsulfosuccinat, Dinatriumlaurylsulfosuccinat und Dinatriumundecylenamido MEA- Sulfosuccinat, Dinatrium PEG-5 Laurylcitratsulfosuccinat und Derivate;
- Schwefelsäureester, wie Alkylethersulfat, beispielsweise Natrium-, Ammonium-, Magnesium-, MIPA-, TIPALaurethsulfat, Natriummyrethsulfat und Natrium C₁₂ bis C₁₃ - Parethsulfat, und Alkylsulfate, beispielsweise Natrium-, Ammoniumund TEA-Laurylsulfat,
- Taurate, beispielsweise Natriumlauroyltaurat und Natriummethylcocoyltaurat;
- Ether-Carbonsäuren, beispielsweise Natriumlaureth-13 Carboxylat und Natrium PEG-6 Cocamide Carboxylat, Natrium PEG-7-Olivenöl-Carboxylat;
- Phosphorsäureester und Salze, wie beispielsweise DEA-Oleth-10 Phosphat und Dilaureth-4 Phosphat;
- Alkylsulfonate, beispielsweise Natriumcocosmonoglyceridsulfat, Natrium C₁₂ bis C₁₄ - Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat;
- Acylpeptide, beispielsweise Palmitoyl hydrolysiertes Milchprotein, Natrium Cocoyl hydrolysiertes Sojaprotein und NatriunWKalium Cocoyl hydrolysiertes Kollagen;
- Carbonsäuren und Derivate, beispielsweise Laurinsäure, Aluminiumstrearat, Magnesiumalkanolat und Zinkundecylenat, Ester-Carbonsäuren, beispielsweise Calciumstearoyilactylat, Laureth-6 Citrat und Natrium PEG-4 Lauramidcarboxylat;
- Alkylarylsulfonate.

Vorteilhafte kationische Tenside im Sinne der vorliegenden Erfindung sind quaternäre Tenside. Quaternäre Tenside enthalten mindestens ein N-Atom, das mit 4 Alkyl-, oder Arylgruppen kovalent verbunden ist. Vorteilhaft sind beispielsweise Alkylbetain, Alkylamidopropylbetain und Alkylamidopropylhydroxysultain.

Weitere vorteilhafte kationische Tenside im Sinne der vorliegenden Erfindung sind ferner Alkylamine, Alkylimidazole und ethoxylierte Amine und insbesondere deren Salze.

Vorteilhafte amphotere Tenside im Sinne der vorliegenden Erfindung sind Acyl-/ dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte aktive nicht-ionische Tenside im Sinne der vorliegenden Erfindung sind Alkanolamide, wie Cocamide MEA/DEA/MIPA, Ester, die durch Veresterung von Carbonsäuren mit Ethylenoxid, Glycerin, Sorbitan oder anderen Alkoholen entstehen, Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil^{®}SPG 128V der Firma Wacker).

Weitere vorteilhafte nicht-ionische Tenside sind Alkohole und Aminoxide, wie Cocoamidopropylamin-Oxid.

Unter den Alkylethersulfaten sind insbesondere Natriumalkylethersulfate auf Basis von zwei oder dreifach ethoxyliertem Laurylund Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut und Schleimhautverträglichkeit. Laurylethersulfat weist bessere Schaumeigenschaften als Myristylethersulfat auf, ist diesem aber in der Milde unterlegen.

Alkylethercarboxylate mit mittlerem und besonders mit höherem gehören zu den mildesten Tensiden überhaupt, zeigen aber ein schlechtes Schaum und Viskositätsverhalten. Sie werden oft in Kombination mit Alkylethersulfaten und amphoteren Tensiden eingesetzt.

Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt.

Amidopropylbetaine weisen eine ausgezeichnete Haut und Augenschleimhautverträglichkeit auf. In Kombination mit anionischen Tensiden lässt sich deren Milde synergistisch verbessern. Bevorzugt ist die Verwendung von Cocamidopropylbetain.

Arnphoacetate/Amphodiacetate besitzen als amphotere Tenside eine sehr gute Haut und Schleimhautverträglichkeit und können konditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind mild, haben gute Universaleigenschaften, schäumen jedoch schwach. Aus diesem Grund werden sie bevorzugt in Kombinationen mit anionischen Tensiden verwendet.

### Konditionierungsmittel

Gegebenenfalls enthalten die erfindungsgemäßen Zusammensetzungen ein Konditionierungsmittel. Bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, SkinConditioning Agents-Emollient, Skin-Conditioning Agents-Humectant, SkinConditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen.

Besondere vorteilhafte Konditionierungsstoffe stellen beispielsweise die nach INCI als Polyquaternium bezeichneten Verbindungen dar (insbesondere Polyquaternium-1 bis Polyquaternium-114).

Zu den geeigneten Konditionierungsmitteln zählen beispielsweise auch polymere quaternäre Ammoniumverbindungen, kationische Cellulosederivate, Chitosanderivate Guar Gum Derivate und Polysaccharide, insbesondere Guar Hydroxypropylammoniumchlorid (z.B. Jaguar^{®} Excel, Jaguar^{®}162 der Firma Rhodia).

Weitere erfindungsgemäß vorteilhafte Konditionierungsmittel stellen nichtionische Poly-N-vinylpyrrolidon/Polyvinylacetat-Copolymere (z.B. Luviskol^{®}VA 64 der Firma BASF AG), anionische Acrylat-Copolymere (z.B. Luviflex^{®}Soft der Firma BASF AG), und/oder amphotere Amid/Acrylat/Methacrylat Copolymere (z.B. Amphomer^{®} der Firma National Starch dar. Weitere mögliche Konditioniermittel sind quaternisierte Silikone.

Optional können herkömmliche Additive ebenfalls in der Zusammensetzung enthalten sein, beispielsweise um ihr bestimmte Modifizierungseigenschaften zu verleihen. Hierbei kann es sich etwa um Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner handeln.

Diese Additive sind im Allgemeinen in einer Konzentration von etwa 0,001 % bis 15 Gew.-% vorzugsweise 0,01 % bis 10 Gew.-% bezogen auf das Gesamtgewicht der Zusammensetzung vorhanden.

In einer bevorzugten Ausführungsform der Haarkosmetischen Zusammensetzung ist mindestens ein Filmbildner der Komponente K3 ausgewählt aus der Gruppe bestehend aus einem nicht-ionischen, einem anionischen, einem amphoteren und/oder einem kationischen Polymer.

Bevorzugt ist der Filmbildner K3 ausgewählt aus der Gruppe bestehend aus einem Polyacrylat, einem Polyacrylamid, einem Polyurethan, einem Polyharnstoff, einem Polysiloxan oder einer Mischung aus mindestens zwei hiervon. Zu den Polyacrylaten werden auch Silikon-Acrylat-Copolymere gezählt.

Unter Anderem zur Verbesserung der Beständigkeit von dekorativen Produkten gegenüber Wasser, Tränen oder Schweiß (oft Wasserfestigkeit genannt) werden filmbildende Polymere als Filmbildner eingesetzt.

Als filmbildende Polymere werden bevorzugt Polymere auf Basis von Acrylaten oder Vinylpyrrolidonen gewählt. Vorteilhafte Filmbildner sind Trimethylsiloxysilicate, Silikon Acrylate Copolymere (z. B. TIB4-200 der Fa. Dow Corning oder KP-561 der Fa. Shin Etsu), Trimethyl Pentaphenyl Trisiloxane (Dow Corning 555 Cosmetic Fluid der Fa. Dow Corning Ltd.) oder Vinylpyrrolidon-Copolymer (z.B. PVP/Eicosen-copolymer oder PVP/hexadecan-copolymer).

Bevorzugt kommen generell nicht-ionische, anionische, amphotere und/oder kationische Polymere als Filmbildner zum Einsatz. Bevorzugt nicht-ionische Polymere, die in der kosmetischen Zusammensetzung entweder alleine oder in Kombination bevorzugt mit anionischen und/oder amphoteren und/oder zwitterionischen Polymeren verwendet werden sind bevorzugt ausgewählt aus der Gruppe bestehend aus:
- Polyalkyloxazoline;
- Vinylacetat Homo- oder Copolymerisate, wobei. hierzu beispielweise Copolymerisate aus Vinylacetat und Acrylsäureester, Copolymerisate aus Vinylacetat und Ethylen, Copolymerisate aus Vinylacetat und Maleinsäureester gehören;
- Acrylsäureester Copolymerisate wie z. B. die Copolymerisate aus Alkyl-Acrylat und AlkylMethacrylat, Copolymerisate aus Alkyl-Acrylat und Urethanen;
- Copolymerisate aus Acrylnitril und nichtionischem monomer ausgewählt aus Butadien und (Meth)Acrylat;
- Styrol Homo- und Copolymerisate, wobei hierzu beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin gehören;
- Polyamide;
- Vinyllactame Homo- oder Copolymere, wie Vinylpyrrolidon-Homo- oder Copolymerisate; wobei hierzu beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat gehören;
- Polysiloxane;
- Homopolymere des N-Vinylformamids wie PVF von AkzoNobel; und
- Polyurethane, wie Baycusan^{®} C2000 von Covestro Deutschland AG.

Besondere bevorzugte nichtionische Polymere sind Acrylsäureester Copolymerisate, Homopolymere des Vinylpyrrolidons und Copolymere sowie Polyvinylcaprolactam.

Besonders bevorzugte nichtionische Polymere sind Homopolymere des Vinylpyrrolidons z. B. Luviskol^{®} K der Firma BASF, Copolymerisate aus Vinylpyrrolidon und Vinylacetat z. B. Luviskol^{®} VA Typen der Firma BASF oder PVPVA^{®} S630L der Firma ISP, Terpolymere aus Vinylpyrrolidon, Vinylacetat und Propionat wie z. B. Luviskol^{®} VAP von BASF und Polyvinylcaprolactame z.B. Luviskol^{®} PLUS der Firma BASF.

Vorteilhafte anionische Polymere sind Homo-oder Copolymere mit Säuregruppen enthaltenden Monomereinheiten, die gegebenenfalls mit Comonomeren, die keine Säuregruppen enthalten copolymerisiert sind. Geeignete Monomere sind ungesättigte, radikalisch polymerisierbare Verbindungen, die mindestens eine Säuregruppe besitzen, und insbesondere Carbonsäure, Sulfonsäure oder Phosphonsäure.

Beispiele für anionische Polymere, die Carbonsäuregruppen enthalten sind:
- Acrylsäure oder Methacrylsäure Homo- oder Copolymere oder deren Salze. Hierzu gehören beispielweise die Copolymerisate aus Acrylsäure und Acrylamide und / oder deren Natriumsalze, Copolymerisate aus Acrylsäure und / oder Methacrylsäure und einem ungesättigten Monomer ausgewählt aus der Gruppe Ethylene, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone^{®} LM der Firma ISP, Copolymerisate aus Methacrylsäure, Ethylacrylate und Tert-butyl Acrylate z.B. Luvimer^{®} 100 P der Firma BASF;
- Crotonsäurederivat-Homo- oder Copolymere oder deren Salze. Hierzu gehören beispielweise Vinylacetat / Crotonsäure-, Vinylacetat/Acrylat- und / oder Vinylacetat / Vinylneodecanoat / Crotonsäure-Copolymere z.B. Resyn^{®} 28-1310 oder Resyn^{®} 28-2930 der Firma AkzoNobel oder Luviset^{®} CAN der Firma BASF, Natriumacrylat/Vinylalkohol-Copolymere;
- ungesättigte C4-C8 Carbonsäurederivate oder Carbonsäureanhydrid Copolymere ausgewählt aus Copolymerisaten aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Allylester, Methallylester und ggfs. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether / Maleinsäureanhydrid-Copolymeren entstehen. Diese Polymere können auch teilverestert (Ethyl, Isopropyl- bzw. Butylester) oder teilamidiert sein;
- in Wasser lösliche oder dispergierbare anionische Polyurethane, z. B. Luviset^{®} P.U.R. der BASF, und Dynamx^{®} von AkzoNobel, Baycusan^{®} C1000, Baycusan^{®} C1001, Baycusan^{®} C1003, Baycusan^{®} C1004, Baycusan^{®} C1008 von Covestro Deutschland AG,
Diese vorangehende Liste ist nicht abschließend und damit nicht einschränkend zu sehen.

Vorteilhafte anionische Polymere enthaltend Sulfonsäuregruppe sind Salze von Polyvinylsulfonsäuren, Polystyrolsulfonsäuren wie z. B. Natriumpolystyrolsulfonat oder von Polyacrylamidesulfonsäuren.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester und Salze von Polystyrolsulfonsäuren.

Ganz besonders vorteilhafte anionische Polymere sind Acrylat-Copolymere, z.B. Luvimer^{®} der BASF, Ethylacrylat / N-tert.-Butylacrylamid / Acrylsäure-Copolymere ULTRAHOLD^{®} STRONG der BASF, VA / Crotonat / Vinylneodecanoat-Copolymer z. B. Resyn^{®} 28-2930 der AkzoNobel, Copolymerisate wie. Z. Copolymerisate aus Methylvinylether und Maleinsäureanhydrid teilverestert z.B. GANTREZ^{®} von Ashland und Natrium-polystyrolsulfonate, wie Flexan^{®} 130 von AkzoNobel.

Vorteilhafte amphotere Polymere können unter den Polymeren ausgewählt werden, die statistisch in der Polymerkette verteilte Einheiten A und B enthalten, wobei A für eine Einheit steht, die von einem Monomer mit mindestens einem basischen Stickstoffatom abgeleitet ist, und B eine Einheit darstellt, die von einem sauren Monomer stammt, das eine oder mehrere Carboxy- oder Sulfonsäuregruppen aufweist. Alternativ können A und B Gruppen sein, die von zwitterionischen Carboxybetainmonomeren oder Sulfobetainmonomeren abgeleitet sind. A und B können auch eine kationische Polymerkette sein, die primäre, sekundäre, tertiäre oder quartanäre Gruppen enthalten, wobei mindestens eine Aminogruppe eine Carboxygruppe oder Sulfonsäuregruppe trägt, die über eine Kohlenwasserstoffgruppe gebunden ist, oder A und Bsind Teil einer Polymerkette mit Ethylen-α,β-dicarbonsäureeinheit, bei der die Carbonsäuregruppen mit einem Polyamin umgesetzt wurden, das eine oder mehrere primäre oder sekundäre Aminogruppen enthält.

Bevorzugte amphotere Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden. Das basische Monomer ist von einer Vinylverbindung abgeleitet, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent US 3,836,537 beschrieben worden.
- Polymere mit Einheiten, die von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat abgeleitet sind.

Besonders bevorzugte N-substituierte Acrylamide oder Methacrylamide sind Verbindun-gen, deren Alkylgruppen 2 bis 12 Kohlenstoffatome enthalten. Ganz besonders bevorzugt sind N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.

Geeignete saure Comonomere sind insbesondere aus der Gruppe Acrylsäure, Methacryl-säure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.

Bevorzugte basische Comonomere sind Aminoethylmethacrylat, Butylaminoethylmethacrylat, N,N-Dimethylaminoethylmethacrylat, N-t-Butylaminoethylmethacrylat..
- Vernetzte und ganz oder teilweise acylierte Polyaminoamide, die von Polyaminoamiden der folgenden allgemeinen Formel:

   -[CO-R-CO-Z]-

   abgeleitet sind, worin R eine zweiwertige Gruppe ist, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bis-sekundäres Amin entsteht, und Z eine Gruppe ist, die von einem bis-primären, mono- oder bis-sekundären Polyalkylenpolyamin abgeleitet ist, und bevorzugt: a) als quantitative Fraktion von 60 bis 100 mol% die Gruppen -NH-[(CH₂)ₓ-NH-]p- wobei x = 2 und p = 2 oder 3 oder x = 3 und p = 2, wobei die Gruppe gebildet wird von Diethylentriamin, Triethylentetramin oder Dipropylentriamin; b) als quantitative Fraktion von 0 bis 40 mol% die Gruppe -NH-[(CH₂)ₓ-NH-]p-, wobei x = 2 und p = 1, das erhältlich ist aus Ethylenediamin, oder der Gruppe, die von Piperazin stammt: 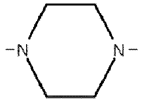
   c) in quantitativer Fraktion von 0 bis 20 mol.-%, die Gruppe -H-(CH₂)₆-NH-, das erhältlich ist aus Hexamethylenediamin, wobei diese Polyaminoamide vernetzt sind durch Addition eines bifunktionalen Vernetzers, bevorzugt ausgewählt aus der Gruppe von Epihalohydrinen, Diepoxiden, Dianhydriden und bis-ungesättigten Derivaten, in einer Menge in einem Bereich von 0.025 bis 0.35 mol von Vernetzer pro Aminogruppe des Polyaminoamids, und acyliert mit Acrylsäure, Chloressigsäure oder einem Alkansulfon oder Salzen davon.

Die gesättigten Carbonsäuren sind vorzugsweise unter den Säuren mit 6 bis 10 Kohlenstoffatomen, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure,

Die bei der Acylierung verwendeten Alkansultone sind vorzugsweise Propansulton oder Butansulton, die Salze der Acylierungsmittel sind vorzugsweise die Natriumsalze oder Kaliumsalze.
- Polymere mit zwitterionischen Einheiten der folgenden Formel: 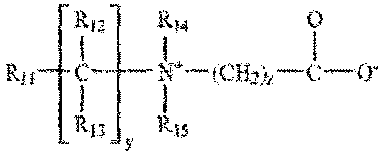 worin R₁₁ eine polymerisierbare ungesättigte Gruppe, wie Acrylat, Methacrylat, Acrylamid oder Methacrylamid, y und z ganze Zahlen von 1 bis 3, R₁₂ und R₁₃ ein Wasserstoffatom, Methyl, Ethyl oder Propyl, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt, sind.

Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 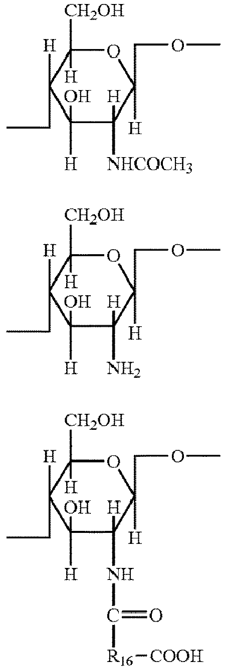 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 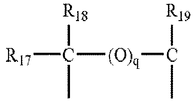 worin falls q = 0, die Gruppen R₁₇, R₁₈ und R₁₉, gleich oder verschieden jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und / oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoffatom, sowie die Salze, die diese Verbindungen mit Basen oder Säuren bilden, sind.
- Polymere, die der folgenden allgemeinen Formel entsprechen und die beispielsweise in dem französischen Patent 1 400 366 beschrieben sind: 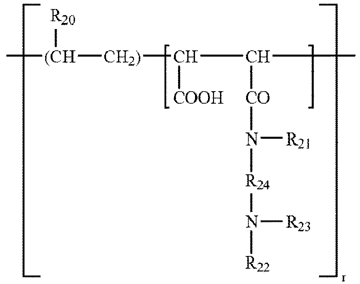 worin R₂₀ ein Wasserstoffatom, CH₃O, CH₃CH₂O oder Phenyl ist, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe, wie Methyl oder Ethyl ist, R₂₃ eine niedere C₁₋₆-Alkylgruppe, wie Methyl oder Ethyl oder eine Gruppe der Formel: -R₂₄-N(R₂₂)₂ ist, wobei R₂₄ eine Gruppe -CH₂-CH₂,-CH₂-CH₂-CH₂- oder -CH₂-CH(CH₃)- darstellt und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.

Alkyl(C₁₋₅)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besonders vorteilhafte amphotere Polymere sind z.B. die Copolymere Octylacrylamid / Acrylate / Butylamino-Ethylmethacrylat-Copolymer, die unter den Bezeichnungen AMPHOMER^{®}, AMPHOMER^{®} LV 71 oder BALANCE^{®} 47 der Firma AkzoNobel im Handel sind, und Methylmethacrylat / Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere, die Säuregruppen enthalten, können folgende Basen eingesetzt werden: Hydroxide, deren Kation Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS) verwendet.

Die Neutralisation kann je nach Anwendungszweck teilweise oder vollständig erfolgen.

Gegebenenfalls einsetzbar aber jedoch weniger bevorzugt sind kationische Polymere, wie beispielweise Polymere, die primäre, sekundäre tertiäre und / oder quaternäre Aminogruppen enthalten, die Teil der Polymerkette oder direkt an die Polymerkette gebunden sind.

[A6] In einer bevorzugten Ausführungsform der Haarkosmetischen Zusammensetzung ist der Filmbildner K3 ein Polyurethan, das erhältlich ist durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23°C weniger als 10 g / Liter, bevorzugter weniger als 5 g / Liter beträgt und das Prepolymer bei 23° keine sedimentationsstabile Dispersion in Wasser, insbesondere entionisiertem Wasser, ergibt. Mit anderen Worten setzt sich das Prepolymer, beim Versuch es in Wasser zu dispergieren, ab.

Bevorzugt ist das NCO-terminierte Polyurethanprepolymer A) erhältlich aus der Reaktion einer Reaktionsmischung umfassend ein Polyisocyanat und Polyol. Das Polyisocyanat weist bevorzugt eine Funktionalität in einem Bereich von >1,5 bis 6, oder bevorzugt von 1,8 bis 5, oder bevorzugt von 2 bis 4, insbesondere von 2 auf. Geeignete Polyisocyanate sind aliphatische, aromatische araliphatische oder cycloaliphatische Polyisocyanate. Beispiele solcher geeigneten Polyisocyanate sind 1,4-Butylendiisocyanat, 1,6-Hexamethy-lendiisocyanat (HDI), Isophorondiisocyanat (IPDI), 2,2,4- und/oder 2,4,4-Trimethyl¬hexa¬methy-len¬¬diiso¬cyanat, die isomeren Bis-(4,4'-isocyanatocyclo¬hexyl)¬methane oder deren Mischungen beliebigen Isomeren¬gehalts, 1,4-Cyclohexylendi¬isocyanat, 4-Isocyanatomethyl-1,8-octandiisocyanat (Nonantriisocyanat), 1,4-Phenylen¬di¬iso¬cya¬nat, 2,4- und/oder 2,6-Toluylendiisocyanat, 1,5-Naphthylen¬diisocyanat, 2,2'-und/oder 2,4'- und/oder 4,4'-Di-phenylmethandiisocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin-diisocyanate) mit C1 bis C8-Alkylgruppen.

Bevorzugt ist das Polyisocyanat ein aliphatisches Polyisocyanat. Bevorzugte aliphatische Diisocyanate sind Hexamethylendiisocyanat und Isophorondiisocyanat sowie deren Gemische.

Das Polyol weist bevorzugt eine Funktionalität von >1,5 bis 6 oder bevorzugt von 1,8 bis 5, oder bevorzugt von 2 bis 4, insbesondere von 2 auf. Bevorzugt ist das Polyol ausgewählt aus der Gruppe bestehend aus einem Polyetherpolyol, einem Polycarbonatpolyol, einem Polyetherpolycarbonatpolyol, einem Polyesterpolyol oder einem Gemisch aus mindestens zwei hiervon. Bevorzugt weist das Polyol ein Polyoxyethylengruppen enthaltendes Polyol auf. Hinsichtlich der Polyole sind Copolymere aus Ethylenoxid und Propylenoxid mit einem Ethylenoxidgehalt, bezogen auf die Gesamtmenge der enthaltenen Oxyalkylengruppen, von 60 bis 85 mol-% bevorzugt. Weiterhin bevorzugt sind Polyesterpolyole bevorzugt gebildet aus einer Säure ausgewählt aus der Gruppe bestehend aus Malonsäure, Bernsteinsäure, Glutarsäure, Adipinsäure, Pimelinsäure, Korksäure, Azelainsäure, Sebacinsäure oder einer Mischung aus mindestens zwei hiervon mit einem Polyol ausgewählt aus der Gruppe bestehend aus 1,2-Ethandiol, 1,3-Propandiol, 2,2-Dimethyl-1,3-propandiol (Neopentandiol), 1,4-Butandiol, 2,2-Dimethyl-1,4-butanediol, 1,5-Pentandiol, 2,2-Dimethyl-1,4-butanediol, 1,6-Hexandiol oder einer Mischung aus mindestens zwei hiervon.

In einer bevorzugten Ausführungsform der Haarkosmetischen Zusammensetzung liegt die Gesamtmenge an Carbodiimiden und die Gesamtmenge an Filmbildnern in einem Verhältnis von 2:1 bis 1:4, oder bevorzugt in einem Verhältnis von 1,5:1 bis 1:3, oder bevorzugt in einem Verhältnis von 1:1 bis 1:3 vor. Bevorzugt weist die kosmetische Zusammensetzung mehr Gewichtsprozent an Filmbildner als an Verbindungen enthaltend mindestens drei Carbodiimidgruppen, auch Polycarbodiimid genannt.

In einer bevorzugten Ausführungsform der Haarkosmetischen Zusammensetzung liegt die Zusammensetzung in Form ausgewählt aus der Gruppe bestehend aus einem Pumpsprays, einem Aerosols, einem Gel, einem Schaum, einem Schaumfestiger, einer Lotion, einem Wachs, einer Pomade, einem Öl, einer Milch, eine Öl in Wasser Emulsion, einer wässrigen Lösung oder einer Creme vor. Die kosmetische Zusammensetzung liegt bevorzugt in Form eines Pumpsprays, eines Aerosols, eines Gels, eines Schaumes, eines Schaumfestiger, einer Lotion, eines Wachs, einer Pomade, vor.

Die erfindungsgemäße Zusammensetzung kann ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Der Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (lkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die Wachse können in Mengen von 0 bis 10 Gew.-%, bezogen auf das Gesamtgewicht der Zusammensetzung und vorzugsweise 0 bis 5 Gew.-% enthalten sein.

Das bevorzugte kosmetische akzeptable Medium der erfindungsgemäßen Zusammensetzung enthält Wasser und gegebenenfalls ein kosmetisch verträgliches in Wasser mischbares geeignetes organisches Lösungsmittel.

Das verwendete Wasser in der erfindungsgemäßen Zusammensetzung kann ein Blütenwasser, reines demineralisiertes Wasser, Mineralwasser, Thermalwasser und/oder Meerwasser sein.

Im Fall einer O/W Zusammensetzung als erfindungsgemäße Zusammensetzung kann der Wasseranteil im Bereich von 40 bis 95 Gew.-%, bevorzugt im Bereich von 50 bis 90 Gew.-%, ganz bevorzugt im Bereich von 60 bis 80 Gew.-% bezogen auf das Gesamtgewicht der Zusammensetzung, liegen. Im Fall einer W/O Zusammensetzung liegt der Wasseranteil im Bereich von 0 bis 60 Gew.-%, bevorzugt im Bereich von 10 bis 50 Gew.-%, ganz bevorzugt im Bereich von 30 bis 50 Gew.-% bezogen auf das Gesamtgewicht der Zusammensetzung.

Die Zusammensetzung kann ferner mit einem Treibgase aufgeschäumt werden. Die oben geschriebenen Emulsionen können durch O/W-, W/O oder W/Si-Emulgator, Verdicker (wie beispielweise Hydrodispersion) oder Feststoffe (wie beispielweise Pickeringemulsion) stabilisiert sein.

Die Zusammensetzung kann einen oder mehrere Emulgatoren bzw. oberflächenaktive Mittel enthalten.

So enthalten insbesondere Öl-in-Wasser-Emulsionen (O/W) bevorzugt mindestens einen Emulgator mit einem HLB-Wert > 7 und gegebenenfalls einen Coemulgator.

Vorteilhaft werden die folgenden nichtionischen Emulgatoren eingesetzt:
•a) Partialfettsäureester und Fettsäureester mehrwertiger Alkohole und deren ethoxylierte Derivate (z. B. Glycerylmonostearat, Sorbitanstearat, Glyceryl-Stearyl-Citrat, Sucrose-Stearat)
•b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/C₁₀₋₃₀-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

In einer bevorzugten Ausführungsform der Haarkosmetischen Zusammensetzung ist die Zusammensetzung zur Behandlung von Haaren, insbesondere zur Strukturerhaltung von Farb-und/oder Formveränderung der Haare, geeignet. Insbesondere wird bei der Behandlung der Haare mit der Haarkosmetischen Zusammensetzung eine weitere haarkosmetische Anwendung vorgenommen. Die weitere haarkosmetische Anwendung kann jede Anwendung an dem Haar sein, die zu einer Farb- und/oder Formveränderung des Haares führt. Durch die Behandlung des Haares mit der erfindungsgemäßen Zusammensetzung wird bevorzugt erreicht, dass das Haar eine höhere Strukturbeständigkeit gegenüber Wasser z.B. in Form eines Wasserstrahls z.B. beim Duschen, Baden oder Schwimmen, oder Haarpflegemitteln wie Shampoo, Haarfestiger etc. erhält, eine geringer Quellung und eine gute Haptik aufweist. Bevorzugt ist die haarkosmetische Anwendung ausgewählt aus der Gruppe bestehend aus
(A) einem Färben mindestens eines Teils der Haare,
(B) einer Dauerwellenbehandlung der Haare,
(C) einer Behandlung mit einem Lockenstab,
(D) einem Glätten der Haare,
(E) einer dauerhaften Glättung der Haare,
(F) einem Frisieren der Haare,
(G) eine Haarverlängerung,
(H) einer Haarverkürzung,
(I) einer Kombination aus mindestens zwei Anwendungen ausgewählt aus (A) bis (H).

Die Behandlung von Haaren mit der erfindungsgemäßen Haarkosmetischen Zusammensetzung findet bevorzugt unter Anwendung von erhöhter Temperatur statt. Die Anwendung von erhöhter Temperatur kann sowohl die zu behandelnden Haare als auch die zu verwendende Haarkosmetische Zusammensetzung oder beides betreffen. Bevorzugt findet die Anwendung von erhöhter Temperatur ausgewählt aus der Gruppe bestehend aus Erwärmung der Haarkosmetischen Zusammensetzung vor Anwendung auf dem Haar, Erwärmung der Haare vor Behandlung mit der Haarkosmetischen Zusammensetzung, Erwärmung der Haare nach Behandlung mit der Haarkosmetischen Zusammensetzung, Erwärmung der Haare während der Behandlung mit der Haarkosmetischen Zusammensetzung oder einer Kombination aus mindestens zwei hieraus statt. Bevorzugt findet die Anwendung von erhöhter Temperatur durch Erwärmen der Haarkosmetischen Zusammensetzung vor Behandlung der Haare mit der Haarkosmetischen Zusammensetzung statt. Die Anwendung von erhöhter Temperatur findet wahlweise vor, während und/oder nach der Behandlung von Haaren mit der erfindungsgemäßen Haarkosmetischen Zusammensetzung statt. Bevorzugt wird das zu behandelnde Haar bei der Anwendung von erhöhter Temperatur mit einem Mittel in Kontakt gebracht, das eine Temperatur in einem Bereich von 30 bis 250 °C, oder bevorzugt in einem Bereich von 40 bis 230 °C, oder bevorzugt in einem Bereich von 50 bis 200 °C, oder bevorzugt in einem Bereich von 60 bis 180 °C aufweist. Bevorzugt findet das Erwärmen der Haarkosmetischen Zusammensetzung vor Behandlung der Haare damit statt, bevorzugt in einem Bereich von 40 bis 120 °C, oder bevorzugt in einem Bereich von 50 bis 110 °C, oder bevorzugt in einem Bereich von 60 bis 100 °C statt. Bevorzugt wird die erhöhte Temperatur dem Haar über erwärmte Luft oder eine erwärmte Oberfläche zugeführt. Die Erhöhung der Temperatur wird bevorzugt durch ein Mittel erreicht ausgewählt aus der Gruppe bestehend aus einem Föhn, einem Glätteisen, einem Lockeneisen, einer Trockenhaube, einem Lockenstab oder einer Kombination aus mindestens zwei hiervon. Die angegebenen Temperaturbereiche für die erhöhte Temperatur ist dabei bevorzugt die Temperatur des Mittels unmittelbar bevor es mit dem Haar in Kontakt gebracht wird.

Die Dauer der Anwendung von erhöhter Temperatur liegt bevorzugt in einem Bereich von 1 Minute bis 3 Stunden, oder bevorzugt in einem Bereich von 5 Minuten bis 2 Stunden, oder bevorzugt in einem Bereich von 10 Minuten bis 1 Stunde, oder bevorzugt in einem Bereich von 20 Minuten bis 50 Minuten.

Ein weiterer Gegenstand der Erfindung betrifft eine Verwendung einer Zusammensetzung, enthaltend mindestens ein Carbodiimid der Formel (I),

R⁴-[R¹-N=C=N]ₙ-R⁵ (I)

wobei
n für eine ganze Zahl von 3 bis 100, bevorzugt von 3 bis 50, oder bevorzugt von 3 bis 20, oder bevorzugt von 3 bis 20 steht,
R¹, R⁴ und R⁵ jeweils unabhängig voneinander für lineare oder verzweigte, aliphatische oder cyclo-aliphatische, gegebenenfalls substituierte Gruppen mit 1 bis 100 C-Atomen, bevorzugt 1 bis 50, oder bevorzugt 1 bis 20 steht,
zur Fixierung von Strukturen auf Haar.

Alle Ausführungen zu der Verbindung enthaltend mindestens ein Carbodiimid, auch Polycarbodiimid genannt, die im Zusammenhang mit der erfindungsgemäßen kosmetischen Zusammensetzung beinhaltend die Verbindung enthaltend mindestens ein Carbodiimid ausgeführt wurden sind bevorzugt entsprechend auf die Verwendung zur Fixierung von Strukturen auf Haar anzuwenden. Unter dem Begriff Fixierung von Strukturen auf dem Haar ist sowohl die Fixierung von künstlichen Strukturen als auch von körpereigenen Strukturen zu verstehen. Beispiele für künstliche Strukturen sind nicht körpereigene oder künstliche Haare, oder sonstige künstliche Elemente, die auf dem Haar aufgebracht werden können. Beispiele für körpereigene Strukturen sind Strukturen, die bereits am Körper des Benutzers vorhanden sind, aber in ihrer Form, Lage oder Ausgestaltung verändert wurden und so fixiert werden sollen, beispielsweise Haarsträhnen oder Teile einer Haarsträhne. Dabei soll unter Fixieren vor allem verstanden werden, dass die gewünschte Form fixiert also über einen längeren Zeitraum erhalten bleiben soll. Die Verwendung der erfindungsgemäßen Haarkosmetischen Zusammensetzung findet bevorzugt unter Anwendung von erhöhter Temperatur statt. Die Anwendung von erhöhter Temperatur findet wahlweise vor, während und/oder nach der Behandlung von Haaren mit der erfindungsgemäßen Haarkosmetischen Zusammensetzung statt. Bevorzugt wird das zu behandelnde Haar bzw. die Haarkosmetische Zusammensetzung vor, bei und/oder nach der Anwendung der Haarkosmetischen Zusammensetzung auf eine Temperatur in einem Bereich von 30 bis 250 °C, oder bevorzugt in einem Bereich von 40 bis 230 °C, oder bevorzugt in einem Bereich von 50 bis 200 °C, oder bevorzugt in einem Bereich von 60 bis 180 °C gebracht. Bevorzugt findet das Erwärmen der Haarkosmetischen Zusammensetzung vor Behandlung der Haare mit dieser in einem Bereich von 40 bis 120 °C, oder bevorzugt in einem Bereich von 50 bis 110 °C, oder bevorzugt in einem Bereich von 60 bis 100 °C statt. Die Dauer der Anwendung von erhöhter Temperatur liegt bevorzugt in einem Bereich von 1 Sekunde bis 3 Stunden, oder bevorzugt in einem Bereich von 5 Sekunden bis 2 Stunden, oder bevorzugt in einem Bereich von 10 Sekunden bis 1 Stunde, oder bevorzugt in einem Bereich von 20 Sekunden bis 10 Minuten.

Ein weiterer Gegenstand betrifft ein kosmetisches Verfahren zum Erzeugen eines dekorativen Effekts auf Haaren, mindestens mit den Schritten:
0) ggf. Färben oder Formen des Haare;
1) Auftragen einer Zusammensetzung, enthaltend mindestens ein Carbodiimid der Formel (I),

   R⁴-[R¹-N=C=N]ₙ-R⁵ (I)

   wobei
   n für eine ganze Zahl von 3 bis 100, bevorzugt von 3 bis 50, oder bevorzugt von 3 bis 20, oder bevorzugt von 3 bis 20 steht,
   R¹, R⁴ und R⁵ jeweils unabhängig voneinander für lineare oder verzweigte, aliphatische oder cyclo-aliphatische, gegebenenfalls substituierte Gruppen mit 1 bis 100 C-Atomen, bevorzugt 1 bis 50, oder bevorzugt 1 bis 20 steht,
   auf das Haar.

Alle Ausführungen zu der Verbindung enthaltend mindestens ein Carbodiimid, auch Polycarbodiimid genannt, die im Zusammenhang mit der erfindungsgemäßen kosmetischen Zusammensetzung beinhaltend die Verbindung enthaltend mindestens ein Carbodiimid ausgeführt wurden sind bevorzugt entsprechend auf das Verfahren zur Anwendung dieser Zusammensetzung auf Haar anzuwenden.

Wie bereits zuvor erwähnt wird unter dekorativem Effekt bereits die Erhaltung von Strukturen der behandelten Haare verstanden. Durch das Verfahren kann bevorzugt auch eine Verstärkung der Strukturen durch die erfindungsgemäße Zusammensetzung ohne weitere Zusatzstoffe wie Pigmente oder Farbstoffe erreicht werden.

Weiterhin ist es bevorzugt, dass durch das Verfahren eine Fixierung von Strukturen auf dem Haar erreicht wird. Unter dem Begriff Fixierung von Strukturen auf dem Haar ist sowohl die Fixierung von künstlichen Strukturen als auch von körpereigenen Strukturen zu verstehen. Beispiele für künstliche Strukturen sind nicht körpereigene oder künstliche Haare, oder sonstige künstliche Elemente, die auf dem Haar aufgebracht werden können. Beispiele für körpereigene Strukturen sind Strukturen, die bereits am Körper des Benutzers vorhanden sind, aber in ihrer Form, Lage oder Ausgestaltung verändert wurden und so fixiert werden sollen, wie Haarsträhnen oder Teile von Haarsträhnen. Dabei soll unter Fixieren vor allem verstanden werden, dass die gewünscht Form fixiert also über einen längeren Zeitraum erhalten werden soll.

Das Färben kann jede optische Veränderung der Haare sein, die der Fachmann hierfür kennt. Das Färben ist bevorzugt ein Färben der Haare mittels entsprechender Haarfärbe- oder Haartönungsmittel. Hierfür können sämtliche kommerzielle Produkte Verwendung finden.

Das Formen des Haares kann jedes Formen sein, dass der Fachmann hierfür auswählen würde. Bevorzugt ist das Formen ausgewählt aus der Gruppe bestehend aus einem Locken, einem Glätten, einem Föhnen, einem Kämmen, einem Bürsten, einem Flechten oder einer Kombination aus mindestens zwei hiervon. Bevorzugt ist das Formen ein Glätten oder ein Locken des Haares. Unter Locken des Haares ist bevorzugt das Ausführen einer Dauerwelle, wie sie überlicherweise beim Frisör durchgeführt wird zu verstehen. Das Glätten kann bevorzugt mit handelsüblichen Glätteisen geschehen und führt selbst bei sehr krausem Haar zum vollständigen Glätten der Haare.

Das Auftragen der Zusammensetzung erfolgt bevorzugt in einer Menge, wie sie üblicherweise für Haarstylingmittel verwendet wird. Bevorzugt wird beim Auftragen eine Menge an kosmetischer Zusammensetzung in einem Bereich von 1 bis 10 ml, bevorzugt in einem Bereich von 2 bis 5 ml verwendet.

Nach dem Auftragen findet ggf. eine Einwirkzeit statt. Die Einwirkzeit beträgt bevorzugt wenige Sekunden bis mehrere Stunden, oder bevorzugt 1 Minute bis 2 Stunden, oder bevorzugt 5 Minuten bis 1 Stunde, oder bevorzugt 10 bis 30 Minuten.

Gemeinsam mit der kosmetischen Zusammensetzung kann ein Filmbildner verwendet werden. Dieser kann sich entweder bereits in der kosmetischen Zusammensetzung befinden oder als weitere Zusammensetzung auf dem Haar aufgetragen werden. Das Auftragen der weiteren Zusammensetzung kann alternativ vor, gleichzeitig oder nach dem Auftragen der kosmetischen Zusammensetzung erfolgen.

In einer bevorzugten Ausführungsform des Verfahrens zum Erzeugen eines dekorativen Effekts auf Haar verbleibt die kosmetischen Zusammensetzung zumindest teilweise darauf, insbesondere auf den aufgetragenen Bereichen des Haares. Insbesondere ist es bevorzugt, dass der dekorative Effekt auch nach mehrmaligem Waschen der Haare erhalten bleibt.

In einer bevorzugten Ausführungsform des Verfahrens zum Erzeugen eines dekorativen Effekts auf Haar wird in einem weiteren Schritt 2) eine weitere Zusammensetzung auf das Haar aufgebracht, die mindestens einen Filmbildner beinhaltet.

Bevorzugt ist der Filmbildner ein Filmbildner wie für die Haarkosmetische Zusammensetzung oben erwähnt. Weiterhin bevorzugt ist der Filmbildner in Schritt 2) ausgewählt aus der Gruppe bestehend aus einem Polyacrylat, einem Polyacrylamid, einem Polyurethan, einem Polyharnstoff, einem Polysiloxan oder einer Mischung aus mindestens zwei hiervon. Bevorzugt ist der Filmbildner in Schritt 2) ein Polyurethan oder ein Polyacrylat. Bevorzugt beinhaltet die weitere Zusammensetzung den Filmbildner in einem Menge in einem Bereich von 0,1 bis 30 Gew.-%, oder bevorzugt in einem Bereich von 0,5 bis 15 Gew.-%, oder bevorzugt in einem Bereich von 1 bis 10 Gew.-%, bezogen auf die Gesamtmasse der weiteren Zusammensetzung. Wie bereits oben erwähnt, kann das Auftragen der weiteren Zusammensetzung vor, gleichzeitig oder nach dem Auftragen der kosmetischen Zusammensetzung erfolgen. Bevorzugt erfolgt das Auftragen der weiteren Zusammensetzung in Schritt 2) vor der Aufbringen der kosmetischen Zusammensetzung in Schritt 1).

In einer bevorzugten Ausführungsform des erfindungsgemäßen Verfahrens findet vor, während und/oder nach den Schritten 1) oder 2) eine Erwärmung des Haares und/oder der Haarkosmetischen Zusammensetzung statt. Das Erwärmen findet bevorzugt vor und/oder während der Behandlung von Haaren mit der erfindungsgemäßen Haarkosmetischen Zusammensetzung in den Schritten 1) oder 2) statt. Bevorzugte Temperaturen zur Erwärmung der Haare oder der Haarkosmetischen Zusammensetzung wurden bereit für die Verwendung der erfindungsgemäßen Haarkosmetischen Zusammensetzung genannt. Bevorzugt wird das zu behandelnde Haar und/oder die Haarkosmetische Zusammensetzung vor und/oder während der Schritte 1) und/oder 2) auf eine Temperatur in einem Bereich von 30 bis 250 °C, oder bevorzugt in einem Bereich von 40 bis 230 °C, oder bevorzugt in einem Bereich von 50 bis 200 °C, oder bevorzugt in einem Bereich von 60 bis 180 °C gebracht. Die Dauer des Erwärmens liegt bevorzugt in einem Bereich von 1 Minute bis 3 Stunden, oder bevorzugt in einem Bereich von 5 Sekunden bis 2 Stunden, oder bevorzugt in einem Bereich von 10 Sekunden bis 1 Stunde, oder bevorzugt in einem Bereich von 20 Sekunden bis 10 Minuten.

In einer bevorzugten Ausgestaltung des Verfahrens zum Erzeugen eines dekorativen Effektes auf Haar beinhaltet weiterhin mindestens einen der folgenden Schritte:
(a) Erwärmen der Haarkosmetischen Zusammensetzung vor dem Auftragen auf das Haar;
(b) Erwärmen der Haare vor Auftragen der Haarkosmetischen Zusammensetzung;
(c) Erwärmen der Haare während des Auftragens der Haarkosmetischen Zusammensetzung;
(d) Erwärmen der Haare nach Auftragen der Haarkosmetischen Zusammensetzung zusammen mit der Haarkosmetischen Zusammensetzung;
(e) Kombination mindestens zwei der Schritte (a) bis (d).

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

Sofern nicht abweichend vermerkt, beziehen sich alle analytischen Messungen auf Messungen bei Temperaturen von 23°C.

Die Bestimmung des pH-Wertes erfolgte mittels einer Elektrode (Gerät Mettler Toledo Seven Excellence).

Das Kämmen fand immer mittels eines Ultron Carbon Line Kamms mit einer Länge von 22,2 cm mit der Seite des Kamms statt, die den breiten Abstand der Kammzinken aufweist. Wobei 3 mal hintereinander durch das Haar gekämmt wurde.

Das Abstreifen der Haare nach Applikation von Zusammensetzungen fand mit Hilfe der Finger statt, in dem alle 5 Finger von oben 3 mal durch die Haarsträhne geführt wurde.

### Vorbereitung bzw. Formen oder Strukturieren von Haarsträhnen (Schritt 0)):

Für alle Tests wurden Euro-Haarsträhnen gewellt (18 cm totale Länge, 8 cm Breite, 1,0 g +/- 0,2 g Gewicht) benutzt. Vor den Tests wurden die Haarsträhnen 1 Minute mit 0,3 g eines kommerziellen Silikonfreien Shampoo (der Firma Syoss "Volume Lift Shampoo") bei 38°C gewaschen. Die Haarsträhnen wurden anschließend 1 Minute bei 38°C ausgespült und mit einem herkömmlichen Kamm (breite Seite) durchgekämmt. Anschließend wurden die Haarsträhnen mit Hilfe eines Föhns 1 Minute bei ca.75°C getrocknet.

Für jedes der unten, sowie in Tabelle 1, aufgeführten Beispiele wurden 3 Haarsträhnen unabhängig voneinander auf die gleiche Weise behandelt. Die in Tabelle 1 aufgeführten Ergebnisse stellen die Durchschnittswerte dieser 3-fach Bestimmung dar.

### Verwendete Substanzen

Die getesteten Substanzen werden mit folgenden Abkürzungen genannt:
- Produkt A:: Zusammensetzung, die gemäß der Vorschrift und den Ausgangmaterialien wie in Beispiel 2 aus der EP 2 371 873 A1 beschrieben, hergestellt wurde;
- Produkt B:: INCI: polyurethane-48, 2 Gew.-%;
- Produkt C:: Amphomer (INCI: Ocylacrylamide/Acrylates/Butyllaminoethyl Methacrylate Copolymer), 100 % mit AMP (Amino Methylpropanol) auf pH 9,6 eingestellt, 2 Gew.-%.

### Schritt 1)

### Vergleichsbeispiel 1:

Nach der Vorbereitung, wie in Schritt 0) beschrieben, wurden die Haarsträhnen erneut mit dem Föhn 3 Minuten bei 75°C getrocknet. Dann wurden die Haarsträhnen 5 mal 3 Sekunde bei 230°C mit einem handelsüblichen Glätteisen geglättet und anschließend 12 Stunden bei 56 % rel.-Luftfeuchtigkeit bei 23 °C vollständig getrocknet.

### Beispiel 2: Behandlung mit Produkt A

Es wurden 3 Haarsträhnen parallel gleich behandelt. Nach der Vorbereitung der Haarsträhnen, wie in Schritt 0 beschrieben, wurde jeweils 1g Produkt A auf die jeweilige Haarsträhne aufgetragen. Jede der drei Haarsträhnen wurde kurz abgestreift, um das Produkt A auf der Länge der jeweiligen Haarsträhne zu verteilen. Die Haarsträhnen wurden gekämmt und mit dem Föhn 3 Minuten bei 75°C getrocknet. Danach wurden sie 5 mal 3 Sekunde bei 230°C mit einem Glätteisen geglättet und anschließend 12 Stunden bei 56 % Luftfeuchtigkeit bei 23 °C vollständig getrocknet.

### Beispiel 3: Behandlung mit Produkt A und anschließend mit Produkt B oder C

Es wurden 3 Haarsträhnen parallel gleich behandelt. Nach der Vorbereitung der Haarsträhnen wie in Schritt 0) beschrieben, wurde jeweils 1 g Produkt A auf jede der drei Haarsträhnen aufgetragen. Jede der drei Haarsträhnen wurde kurz abgestreift, um das Produkt A auf der Länge der Haarsträhne zu verteilen, gekämmt und mit dem Föhn 1 Minute bei 75°C getrocknet.

Anschließend wurde auf jede der drei Haarsträhnen 1 g von Produkt B oder Produkt C auf die jeweilige Haarsträhne aufgetragen. Die Haarsträhnen wurden kurz abgestreift, gekämmt und mit dem Föhn 3 Minute bei 75°C getrocknet.

Anschließend wurden die Haarsträhnen 5 mal 3 Sekunde bei 230°C mit einem Glätteisen geglättet und anschließend 12 Stunden bei 56 % Luftfeuchtigkeit bei 23 °C vollständig getrocknet.

### Beispiel 4: Behandlung mit Produkt Produkt B oder C und Produkt A

Drei gleiche Haarsträhnen wurden wie im Beispiel 3 beschrieben bearbeitet, mit dem Unterschied, dass die Reihenfolge des Aufbringens der Produkte A und der Produkte B oder C umgekehrt wird, also das jeweilige Produkt B oder C zuerst auf die Haarsträhnen appliziert wird, und danach das Produkt A.

### Beispiel 5: Behandlung mit einer Mischung aus Produkt A und Produkt B oder C

Drei gleiche Haarsträhnen wurden nach der Vorbereitung, wie in Schritt 0) beschrieben, werden 1g Produkt A und 1g Produkt B oder Produkt C auf die Haarsträhne aufgetragen. Die Haarsträhnen werden kurz abgestreift, gekämmt und mit dem Föhn 3 Minuten bei 75°C getrocknet. Danach werden sie 5 mal 3 Sekunde bei 230°C mit einem Glätteisen geglättet und anschließend mehrere Stunden bei 56% Luftfeuchtigkeit bei Raum Temperatur vollständig getrocknet.

### Prüfung der Wasserbeständigkeit

Jede der in den Beispielen 1 bis 5 behandelten Haarsträhnen wurden einzeln für 30 Sekunden vollständig in ein auf 38 °C beheiztes Wasserbad mit 2 Liter Inhalt getaucht, für 10 Sekunden abtropfen gelassen und bei Raum Temperatur an Luft vollständig für 24 h trocknen lassen. Die Breite der Strähne wurde anschließend mit einem Lineal (1 Millimeterskala) vermessen.

### Prüfung der Waschbeständigkeit:

Die Haarsträhnen aus Schritt 1. wurden 1 Minute mit 0,3g des kommerziell erhältlichen, Silikonfreien Shampoo (Syoss Volume Lift Shampoo) bei 38°C gewaschen. Die Haarsträhnen wurden anschließend 1 Minute in ein auf 38°C beheiztes Wasserbad für 30 Sekunden ausgespült und mit einem Kamm (breite Seite) durchgekämmt. Anschließend wurden die Haarsträhnen bei Raumtemperatur für 24 h getrocknet. Die Breite der Strähne wurde anschließend mit einem Lineal (1 Millimeterskala) vermessen.

### Ergebnisse:

Die Ergebnisse sind in der Tabelle 1 zusammengefasst.
Beispiel 1 : Behandlung ohne Produkt (Vergleichsbeispiel)
Beispiel 2-1 : Behandlung mit Produkt A (erfindungsgemäß)
Beispiel 3-1 : Behandlung mit Produkt A und Produkt B (erfindungsgemäß)
Beispiel 3-2 : Behandlung mit Produkt A und Produkt C (erfindungsgemäß)
Beispiel 4-1 : Behandlung mit Produkt B und Produkt A (erfindungsgemäß)
Beispiel 4-2 : Behandlung mit Produkt C und Produkt A (erfindungsgemäß)
Beispiel 5-1 : Behandlung mit einer Mischung aus Produkt A und Produkt B (erfindungsgemäß)
Beispiel 5-2 : Behandlung mit einer Mischung aus Produkt A und Produkt C (erfindungsgemäß)

**Tabelle 1: getestete Zusammensetzungen**

| Beispiel | Eingesetzte Produkte | Breite vor / nach Glättung [cm] | Breite nach Wasserbeständigkeitsprüfung [cm] | Breite nach Waschbeständigkeitsprüfung [cm] | |
|---|---|---|---|---|---|
| | | | | 1 mal gewaschen | 2 mal gewaschen |
| 1 | - | 8 / 1,4 | 5 | > 9 | > 9 |
| 2-1 | A | 8 / 1,4 | 1,7 | 3,5 | 4,4 |
| 3-1 | A dann B | 8 / 1,4 | 2,1 | 1,7 | 1,9 |
| 3-2 | A dann C | 8 / 1,4 | 2,3 | 8,7 | - |
| 4-1 | B dann A | 8 / 1,4 | 1,4 | 1,7 | 2,4 |
| 4-2 | C dann A | 8 / 1,4 | 4,1 | 7,3 | - |
| 5-1 | Gemisch A + B | 8 / 1,4 | 1,3 | 2,0 | 2,4 |
| 5-2 | Gemisch A + C | 8 / 1,4 | 3,1 | 7,3 | 7,5 |

Wie in Tabelle 1 zu erkennen ist, hat die Behandlung von Haarsträhnen durch das Produkt A (Beispiel 2-1), das eine erfindungsgemäße Polycarbodiimidzusammensetzung darstellt, einen strukturerhaltenden Effekt auf die behandelten Haarsträhnen, da die Breite der Haare auch nach der Behandlung in Wasser sowie der Behandlung mit Shampoo zu einer geringeren Verbreiterung der Haarsträhne führte als ohne die Anwendung von Carbodiimiden oder unter Anwendung anderer nicht erfindungsgemäßer Zusammensetzungen.

Lediglich eine Verbreiterung um das 1,21 fache der behandelten, also geglätteten Haarsträhnen nach der Wasserbehandlung ist in Beispiel 2-1 zu verzeichnen. Die anschließende Waschung der Haarsträhnen mit Shampoo führte im ersten Waschschritt zu einer Verbreiterung der ursprünglichen Haarsträhne um das 2,5-fache und im zweiten Waschschritt um das 3,14-fache. Die besten Ergebnisse im Sinne einer Strukturerhaltung der geglätteten Haare wurden mit Hilfe einer Kombination des Produktes A mit dem Produkt B erreicht. Sowohl in der Kombination beider Produkte (Beispiel 5-1) in einem Schritt als auch in der nacheinander erfolgten Anwendung von Produkt A und dann Produkt B (Beispiel 3-1) konnten im Vergleich zu Kombinationen mit Produkt C und im Vergleich zu der alleinigen Anwendung mit Produkt A die besten Ergebnisse erzielt werden, wie in Tabelle 1 zu erkennen ist.

Im Gegensatz hierzu verbreitern sich die nichtbehandelten Haare aus dem Vergleichsversuch 1, die mit keinem der Produkte A, B oder C behandelt wurden, eine Verbreiterung um das 3,5 fache erfahren und damit breiter waren als alle anderen Beispiele nach der Waschung. Eine Glättung der Haare mit einem Glätteisen führt folglich ohne strukturerhaltende Maßnahmen bereits nach einer Waschung annähernd wieder zu dem ursprünglich gelockten Haar, während die Behandlung mit einer erfindungsgemäßen Zusammensetzung zumindest bei Wasserkontakt zu einem strukturerhaltenden Effekt führt. Das Waschen mit dem Shampoo ergab eine Verbreiterung des Haares zu einem größeren Wert als dem Ausgangswert vor dem Glätten.

## Patentansprüche

1. Eine Haarkosmetische Zusammensetzung, beinhaltend mindestens eine Verbindung enthaltend mindestens drei Carbodiimidgruppen aufgebaut entsprechend der Formel (I),
R⁴-[R¹-N=C=N]ₙ-R⁵ (I)
wobei
n für eine ganze Zahl von 3 bis 100 steht,
R¹, R⁴ und R⁵ jeweils unabhängig voneinander für lineare oder verzweigte, aliphatische oder cyclo-aliphatische, gegebenenfalls substituierte Gruppen mit 1 bis 100 C-Atomen steht.

2. Die Haarkosmetische Zusammensetzung gemäß Anspruch 1, wobei die Verbindung enthaltend mindestens drei Carbodiimidgruppen die allgemeine Formal (II) aufweist: 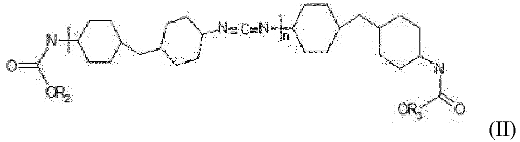 worin R₂ und R₃, unabhängig voneinander, für einen von einer Verbindung ausgewählt aus der Gruppe bestehend aus einem Monoalkoxy-poly(ethylenglykol) gemäß der allgemeinen Formel (III)
R⁶-O-(CH₂-CH₂-O)ₘ-H (III)
mit R⁶ = C₁ bis C₃₀ Alkyl oder Acylgruppe und m = 4 bis 30
und einem von C₁ bis C₃₀ Alkohol oder einem C₁ bis C₃₀ Monoalkoxyethylenglykol abgeleiteten Rest stehen.

3. Die Haarkosmetische Zusammensetzung gemäß einem der vorhergehenden Ansprüche, wobei die Zusammensetzung die Verbindung enthaltend das Carbodiimid der Formel (I) oder (III) in einem Bereich von 0,01 bis 50 Gew.-% enthält.

4. Die Haarkosmetische Zusammensetzung gemäß einem der vorhergehenden Ansprüche, wobei die Zusammensetzung weiterhin mindestens eine der folgenden Komponenten beinhaltet:
1) mindestens eine kosmetisch aktive Substanz und/oder ein oder mehrere kosmetische Hilfssubstanzen,
2) mindestens ein Lösungsmittel oder Verdünner,
3) mindestens einen Filmbildner.

5. Die Haarkosmetische Zusammensetzung nach dem vorhergehenden Anspruch, wobei mindestens einer der Filmbildner aus Komponente K3 ausgewählt ist aus der Gruppe bestehend aus einem einem nicht-ionischen, einem anionischen, einem amphoteren und/oder einem kationischen Polymer oder einer Mischung aus mindestens zwei hiervon.

6. Die Haarkosmetische Zusammensetzung gemäß einem der beiden vorhergehenden Ansprüche, wobei der Filmbildner K3 ein Polyurethan ist, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

7. Die Haarkosmetische Zusammensetzung nach einem der vorhergehenden Ansprüche, wobei die Gesamtmenge an Carbodiimiden und die Gesamtmenge an Filmbildnern in einem Verhältnis von 2:1 bis 1:4 vorliegt.

8. Die Haarkosmetische Zusammensetzung nach einem der vorhergehenden Ansprüche, wobei die Zusammensetzung in Form ausgewählt aus der Gruppe bestehend aus einem Pumpsprays, einem Aerosols, einem Gel, einem Schaum, einem Schaumfestiger, einer Lotion, einem Wachs, einer Pomade, einem Öl, einer Milch, einer Emulsion, einer wässrigen Lösung oder einer Creme vorliegt.

9. Die Haarkosmetische Zusammensetzung nach einem der vorhergehenden Ansprüche zur Behandlung von menschlichen Haaren, insbesondere zur Erhaltung von künstlich geformten Strukturen des Haares.

10. Verwendung einer Zusammensetzung, enthaltend mindestens ein Carbodiimid der Formel (I),
R⁴-[R¹-N=C=N]ₙ-R⁵ (I)
wobei
n für eine ganze Zahl von 3 bis 100 steht,
R¹, R⁴ und R⁵ jeweils unabhängig voneinander für lineare oder verzweigte, aliphatische oder cyclo-aliphatische, gegebenenfalls substituierte Gruppen mit 1 bis 100 C-Atomen steht,
zur Fixierung von Strukturen auf Haaren.

11. Ein kosmetisches Verfahren zum Erzeugen eines dekorativen Effekts auf Haar mindestens mit den Schritten,
0) Ggf. Färben oder Formen des Haares,
1) Auftragen einer Zusammensetzung auf das Haar, wobei die Zusammensetzung mindestens ein Carbodiimid der Formel (I) beinhaltet,
R⁴-[R¹-N=C=N]ₙ-R⁵ (I)
wobei
n für eine ganze Zahl von 3 bis 100 steht,
R¹, R⁴ und R⁵ jeweils unabhängig voneinander für lineare oder verzweigte, aliphatische oder cyclo-aliphatische, gegebenenfalls substituierte Gruppen mit 1 bis 100 C-Atomen steht,
auf das Haar.

12. Das Verfahren nach Anspruch 11, wobei die Zusammensetzung nach dem Auftrag auf das Haar zumindest teilweise auf diesem verbleibt.

13. Das Verfahren nach einem der beiden Ansprüche 11 oder 12, wobei in einem weiteren Schritt 2) eine weitere Zusammensetzung auf das Haar aufgebracht wird, die mindestens einen Filmbildner beinhaltet.

14. Das Verfahren nach einem der Ansprüche 11, 12 oder 13, wobei der Schritt 2) vor dem Schritt 1) erfolgt.

15. Das Verfahren nach einem der Ansprüche 11 bis 14, wobei vor, während und/oder nach den Schritten 1) oder 2) eine Erwärmung des Haares stattfindet.
